Europäisches Patentamt

(19) European Patent Office

Office européen des brevets

(11) Publication number: **0 086 071**
**B1**

# EUROPEAN PATENT SPECIFICATION

(45) Date of publication of patent specification: **06.11.85**

(21) Application number: **83300483.1**

(22) Date of filing: **31.01.83**

(51) Int. Cl.[4]: **A 61 K 39/00,** A 61 K 39/145, C 12 N 7/06

(54) Ozone inactivated organisms as vaccines.

(30) Priority: **09.02.82 US 348569**

(43) Date of publication of application:
**17.08.83 Bulletin 83/33**

(45) Publication of the grant of the patent:
**06.11.85 Bulletin 85/45**

(84) Designated Contracting States:
**AT BE CH DE FR GB IT LI LU NL SE**

(56) References cited:
**BE-A- 471 250
CH-A- 173 194
CH-A- 200 180
GB-A-1 230 414**

**BACTERIOLOGICAL PROCEEDINGS, vol. 167,
1980, page 208 D.C. BOLTON et al.: "Biological
effects of ozone aerosols on five groups of
animal viruses"**

(73) Proprietor: **THE REGENTS OF THE UNIVERSITY
OF CALIFORNIA
2200 University Avenue
Berkeley, California 94720 (US)**

(72) Inventor: **Zee, Yuan Chung
1015 Kent Drive
Davis California, 95616 (US)**
Inventor: **Bolton, David Cecil
2283 9th Avenue
San Francisco California 94116 (US)**

(74) Representative: **Harrison, David Christopher
et al
MEWBURN ELLIS & CO 2/3 Cursitor Street
London EC4A 1BQ (GB)**

(56) References cited:
**PROCEEDINGS OF THE SOCIETY FOR
EXPERIMENTAL BIOLOGY AND MEDICINE, vol.
53, 1943, pages 71-73 J.F. KESSEL et al.:
"Comparison of chlorine and ozone as virucidal
agents of polyomyelitis virus"
CHEMICAL ABSTRACTS, vol. 89, 1978, page
311, no. 220378g, Columbus, Ohio, USA I.N.
SHLYGINA et al.: "Use of ozone for disinfection
of waste water from biological enterprises"**

WATER RESEARCH, vol. 10, 1976, pages 629-631, Pergamon Press, GB. E. KATZENELSON et al.: "Disinfection of viruses in sewage by ozone"
Pascal "Nouveau Traité de Chimie Minérale", Vol. XIII, p. 303 (1960) (Ed. Masson)

## Description

The immune system is responsive to a wide variety of invading microorganisms. However, the response of the mammalian immune system may be relatively slow so that the invading organisms become established, resulting in disease symptoms which may include non-regenerative destruction of host functions. It is found that the response to an invading organism can be greatly accelerated by exposing the host to antigens specific for the invading microorganism. One difficulty is in providing antigen associated with the microorganism in a form which will sufficiently activate the immune system, so that upon subsequent exposure to the live microorganism, the immune system will be effectively galvanized. Other concerns include the completeness of the inactivation of the microorganism, precluding the potential for complementation, and the potential for producing toxic or leaving toxic residues as a result of the inactivation treatment.

One approach to producing vaccines is to inactivate the natural organism. In inactivating the natural organism, it is essential that the inactivation be complete, for residual live organisms could multiply in the host and cause the disease from which the host is to be protected. In addition, the inactivated microorganism must retain its immunogenic morphology to ensure the activation of the immune response to the native antigens. In addition, one wishes to maintain the immunogenic stress to ensure a strong humoral immune response.

Psoralens have been taught for inactivating microorganisms for use as vaccines in U.S. Patent Nos. 4,124,598 and 4,196,281. Bolton et al., Abstract entitled "Biological Effects of Ozone Aerosols on Five Groups of Animal Viruses" of a paper presented at the American Society of Microbiology, which was published in Bacteriological Proceedings (1980) 167:280. Various theories concerning ozone inactivation have been reported. See for example Chan et al. (1977) J. Biol. Chem. 252:8537—8541; Meiners et al. (1977) Environ. Res. 14:99—112; and Freeman et al. (1979) Arch. Biochem. Biophys. 197:264—272. Ozone inactivation of viruses has also been described by Kessel et al., Soc. for Exp. Biol. Med. Proc. (1943) 53:71—73 and Katzenelson and Bidermann, Water Research (1976) 10:629—631. Pascal et al., however, mentioned in the "Nouveau traité de Chimie Minérale", Vol. 13, p. 303 (1960) that ozonisation may lead to a reinforcement of the activity of certain microorganisms whereas other are completely destroyed hence also may lose their immunogenicity. In the invention, viruses, particularly enveloped viruses, are treated with ozone for sufficient time to inactivate the virus to provide a vaccine. The inactivated viruses are then formulated in conventional ways and administered to a host. The vaccines are found to activate the immune system to protect the host from a subsequent infection by the live viruses.

Method and compositions are provided for preparing vaccines and using the vaccines to protect a host from infection by the live organism. The vaccine is prepared by treating the live virus in an appropriate medium with ozone under conditions where it is inactivated. The treated virus may then be stored, particularly at reduced temperatures, or lyophilized, to ensure the retention of its immunogenicity. Prior to use, the inactivated virus may be formulated in accordance with conventional techniques and administered to the host in a variety of ways. As appropriate, additional administrations or booster shots may be employed to provide an enhanced immune response upon invasion by the live virus.

A wide variety of virus may be inactivated, to some degree, with varying effect. The viruses, may be specific for a wide variety of mammals, particularly humans and domestic animals, e.g., bovine, porcine, equine, and ovine.

A wide variety of viruses may be inactivated either enveloped with a lipid bilayer or non-enveloped. Included among the enveloped viruses are members of the Orthomyxoviridae e.g. influenza A virus, members of the Rhabdoviridae, such as vesicular somatitis virus members of the Herpes viridae, such as infectious bovine rhinotracheitis virus, and members of Paramyxoviridae, Togaviridae, Coronaviridae and Reoviridae.

The non-enveloped viruses appear to have greater resistance to ozone inactivation and may require incorporation of the viruses into a liposome to provide the lipid bilayer. Methods for encapsulating various compositions into liposomes to provide a lipid bilayer coating are well known in the literature. The virions may be isolated from tissue cell culture and combined with lipophilic and amphiphilic compounds, such as phospholipids e.g. aliphatic carboxylic acids, of 10 to 22 carbon atoms esterified with phosphatidyl choline, phosphatidyl ethanolamine, phosphatidyl serine, phosphatidic acid, etc. sphingomyelin, cardiolipin, cholesterol, lecithin and galactocerebroside. For preparing liposomes, see, for example, Vemura and Kinsky, Biochemistry (1972) 11:485 and Six et al. *ibid* (1973) 12:403.

In inactivating the viruses with ozone the inactivation should be complete without significant modification of morphology, particularly antigenic recognition sites. Otherwise, the vaccine may result in an immune response to antigenic determinant sites which will not be effective in enhancing the response to the native or wild type virus.

The viruses are initially grown in an appropriate medium. Depending upon the nature of the pathogen, various nutrient media and various techniques may be employed for the propagation of the microorganism. For viruses, the viruses will normally be grown in appropriate tissue culture at a relatively low multiplicity, e.g. 0.01—2 pfu/cell. The organisms may then be freed of cellular debris, titrated for active virus, and stored for usage or used immediately.

The virus infected cells or viral suspensions may then be transferred to the ozone inactivator. Various inactivators may be used which provide for large surface area contact between the ozone containing gas and the viruses to be inactivated. The method should provide substantial contact between the gas and liquid.

One inactivator provides means for maintaining a thin film of the virus dispersion, particularly a thin film whose surface can be continuously changed, ensuring that all of the solution has direct exposure to the ozone stream. This type of exposure is conveniently achieved by using rotating vessels, e.g. roller bottles.

The bottles are conveniently rotated at a slow rate during exposure, generally from about 0.1 to about 20 rpm. This exposure permits the ozone to react with samples with only a thin film of fluid over most of the bottle surface. An ozone source is employed and the ozone diluted into an oxygen containing humidified gas stream e.g. air. Generally, the ozone concentration is maintained between about 0.05 ppm and about 10 ppm, more usually 1 to 5 ppm. The flow rates will generally vary from about 0.5 to 10 l/minute, more usually from about 1 to 8 l/minute, preferably about 3 to 6 l/minute.

Another inactivator provides for the dispersion of small bubbles, e.g. microbubbles, into an agitated suspension of the viruses in a nutrient medium. Conveniently, a fritted disk or other dispersion means may be employed for providing a continuous stream of ozone containing gas bubbles through the nutrient medium. In addition to the agitation resulting from the bubbles, mechanical agitation may also be employed. The other conditions will be analogous to the conditions indicated above.

A wide variety of nutrient media may be employed during the ozone inactivation. Illustrative media include Hank's medium, Eagle's minimal essential medium, and Dulbecco's modified minimum essential medium. Also, included in the above media may be fetal calf serum at about 1 to 5%, buffers and antiobiotics, as illustrated by Hepes buffer at about 5—20 mM and aminoglycosides such as gentamicin, at about 25—75 µg/ml.

The volume of the virus suspension introduced into the ozone inactivation vessel should provide a film of less than about 2 mm in thickness, preferably less than about 1 mm in thickness, assuming the vessel is evenly coated. The concentration of the virus will generally range from about $10^7$ to $10^{10}$, preferably $10^8$ to $10^{10}$ pfu/ml.

Times of exposure may vary widely, depending upon the nature of the particular virus, the amount of ozone in the stream, the sensitivity of the virus to the ozone, and the temperature. The time will be sufficient to ensure the complete inactivation of the virus without significant modification of its morphology. Times will generally be at least about 1 hour, and not more than about 96 hours, generally ranging from about 12 hours to 72 hours. With each virus, the optimum time will be determined empirically.

Either reduced or elevated temperatures may be employed, generally ranging from about 10 to 50°C, more usually from about 20 to 40°C, preferably at mildly elevated temperatures, namely from about 30 to 40°C.

After exposing the virus to the ozone, the inactivated virus may then be isolated in accordance with conventional ways. The exposed viruses are removed from the bottles, and may be isolated by any convenient separation technique, e.g. mild centrifugation. The viruses may then be screened to determine whether inactivation has been complete. Alternatively, radiolabeled nutrients may be employed to determine the presence of incorporation of the radiolabeled nutrients into the virus. Once it has been established that the viruses are totally inactivated, they may then be formulated in appropriate formulations for administration as a vaccine.

The vaccines may be formulated in a wide variety of ways, as solid or liquid formulations and as aerosols. Administration may be orally or by injection, for example, subcutaneously and intravenously. Alternatively, the vaccine may be administered as a spray, so as to be taken nasopharyngeally.

Depending upon the manner of administration, and the host to be vaccinated, the dosage of the inactivated organism may vary widely. For viruses, for example, there can be from $10^6$ to $10^7$ pfu/dose for mice, with the amount for larger mammals increased relative to the weight, but not in direct proportion. That is, for a human, the dosage would be increased in the range of about 10 to 100 times.

Depending upon the host, the pathogen, the manner of administration and the medium in which the ozone-inactivated pathogen is administered, the amount of pathogen will vary widely. For injection, generally about 0.05 ml to 2 ml will be injected. Different pathogens are measured in different units based on particular biological tests. For testing, 1:100 and 1:10 dilutions are employed to determine hypersensitivity. Dosages will generally vary from about $10^6$ viruses to $10^9$ viruses for adult humans, while children will generally be given about one-half the dosage. With each virus optimum amounts will be determined empirically.

Single or multiple administrations may be involved. Where multiple administrations are involved, they will normally be spaced from about 2 to 8 weeks apart, more usually from about 3 to 6 weeks apart.

The following examples are offered by way of illustration and not by way of limitation.

Experimental

Ozone Exposure System: The exposure system was designed and constructed to permit long-term exposure of cell cultures or virus suspensions to ozone. The system accommodated two vessels for ozone exposure and one control vessel. To prevent the reaction of ozone with the non-biological components of the system, all of the system components which would come into contact with ozone were made of the

4

inert materials glass, Teflon, or stainless steel. Compressed air (3.5 kg/cm²) was introduced into the system through a pressure regulator set at 141 g/cm² and filtered through·two ultrafilters (Mine Safety Appliances Co., Pittsburgh, PA) in series. Each ultrafilter was rated at 99.99% efficiency for particles of 0.3 μm diameter and contained a supplemental charcoal element. Ozone at approximately 2 ppm was introduced into the system and mixed with the filtered air using two regulating valves to adjust the ozone concentrations. The main flow of the gas was directed into a 37°C incubator which housed the humidifying bubblers, the roller apparatus, and the sequential sampler. The flow rate of gas through each exposure vessel was held constant by diverting around the sequential sampler a flow rate of gas equal to that sampled. The biological material to be exposed was in sterile 11 cm×29 cm borosilicate glass roller bottles (New Brunswick Scientific, New Brunswick, NJ) equipped with specially machined roller caps with Viton seals. The bottles were rotated at a rate of 1.5 rpm during exposure to permit the ozone to react with the samples with only a thin film of fluid over most of the bottle surface. Ozone concentrations were measured immediately before entering and after exiting the exposure vessel. The gas was dehumidified by cooling in an ice bath before ozone measurement or before entering the downstream flow rotameters.

Ozone Generation and Monitoring: Ozone was generated in medical grade oxygen by silent electric arc discharge wtih a Sander model IV ozoniser (Erwin Sander Elektroapparatebau G.m.b.H. and Co., Am Osterberg, W. Germany) regulated by a constant voltage power supply. Ozone concentrations were measured at flow rates of 3 L/minute.

Propagation and Titration of Virus: Animal virus were propagated at 37°C in monolayers of susceptible cells (described below) grown in roller culture. The medium used for virus production was either Dulbecco's modified Eagle's minimal essential medium supplemented with nonessential amino acids (DEMA) or Eagle's minimal essential medium (MEM). Both media contained 12.5 μg/ml gentamicin, 12.5 $m$M HEPES buffer, and 1 to 2% fetal calf serum (FCS) (Sterile Systems, Logan, UT). The infected cells and supernatant fluids were collected and stored at −70°C until clarified of cellular debris by centrifugation at 10 krpm in the Sorvall GSA rotor for 20 minutes at 4°C. The resultant supernatants were stored in aliquots at −70°C until exposed to ozone.

Influenza A virus (WSN strain) was grown in Madin-Darby bovine kidney (MDBK) cells. The cells were infected at a multiplicity of 0.01 plaque-forming units (Pfu) per cell and incubated for 27 hours.

Exposure of Virus Suspensions to Ozone: The cell culture/virus exposure system described above was used to expose the virus suspensions to ozone. Two hundred and fifty ml aliquots of the virus suspension to be exposed were placed in three 11 cm by 29 cm borosilicate glass roller culture bottles equipped with specially-designed caps. The bottles were connected to the exposure system and rotated at 1.5 revolutions per minute at 37°C while the humidified gas (0.64 ppm ozone) flowed through the bottles at a rate of 6 L/minute. Ozone reacted with the virus suspended in a thin film of fluid on the surface of the rotating culture bottles. The suspension was exposed for 72 h.

In order to demonstrate the effectiveness of the inactivated vaccines, a test was carried out with 63 mice divided into approximately four groups. The ozone-inactivated influenza virus was formulated in a physiologically acceptable buffered medium and administered in a variety of ways. The following indicates the protocols and the results.

Sixty-three seven week old Swiss-Webster female mice were divided approximately into 4 groups. The medium is tissue culture medium with 5% FCS, containing $5\times10^6$ Pfu per 0.2 ml.

Group I (15 mice) received 0.2 ml of ozone-inactivated influenza virus vaccine subcutaneously.

Group II (15 mice) received 0.2 ml of vaccine subcutaneously and received an additional booster of 0.2 ml of vaccine 2 weeks later after the initial vaccine injection.

Group III (15 mice) received 0.1 ml of vaccine intranasally.

Group IV (18 mice) is the control group and did not receive any vaccine.

All the groups were challenged with an aerosol of influenza virus 3 weeks later after the vaccination. Mortality rate and clinical symptoms were recorded each day after influenza virus challenge. Results were recorded in the following table.

TABLE I

| | #Mice died / #Mice used | Mortality rate |
|---|---|---|
| Group I | 0/15 | 0% |
| Group II | 2/15 | 13% |
| Group III | 0/15 | 0% |
| Group IV | 13/18 | 70% |

To further demonstrate the subject invention, a more extended test was carried out. In the test, 140 10-week old female Swiss-Webster mice were divided into two equal groups, one of the groups received 0.2 ml of vaccine (see above) subcutaneously (Group I) and the other group received 0.2 ml placebo (ozone-inactivation media) subcutaneously (Group II). Two mice from Group I after vaccination are bled on the following days (0, 6, 12, 20) and pooled sera are titrated for neutralizing antibody (NA) by the 80% plaque reduction method. Both groups are exposed to an influenza aerosol ($2\times10^6$ pfu/ml on the 21st day. Four mice from each group are removed on days 1, 2, 4, 6, 8 and 12 after viral challenge; their lungs are collected for virus titration and the sera pooled for NA test. Forty mice from each group are kept for mortality data.

The following tables indicate the results.

TABLE II

| | #Mice died / #Mice used | Mortality rate % |
|---|---|---|
| Group I (vaccinated) | 1/38 | 2.6 |
| Group II (control) | 14/40 | 35.0 |

TABLE III

| Days post-infection | Virus titer in 10% lung homogenate (pfu/ml) Group I | Group II |
|---|---|---|
| 1 | $9.4\times10^4$ | $3.6\times10^6$ |
| 2 | $1.1\times10^4$ | $1.0\times10^7$ |
| 6 | $<10^3$ | $3.7\times10^7$ |
| 8 | $<10^2$ | $9.5\times10^5$ |
| 12 | $<10^2$ | $3.0\times10^2$ |

TABLE IV

| Days after vaccination | Serum neutralizing antibody titer 80% plaque reduction Group I | Group II |
|---|---|---|
| 0 | 0 | |
| 6 | 0 | |
| 13 | 0 | |
| 20 | 1:128 | |
| Days after influenza challenge | | |
| 1 | 1:32 | 0 |
| 2 | 1:128 | 0 |
| 6 | 1:512 | 1:8 |

Table II showed that the vaccine was effective in the protection of mice against influenza virus challenge. Table III showed that the influenza virus titers in the lung homogenates of vaccinated mice were approximately two to four logs lower than the control mice from one day post-infection to eight days post-infection. Table IV indicates the serum neutralizing titers (80% plaque reduction) in Group I mice after receiving the subcutaneous influenza vaccine and in both groups of mice after aerosol influenza virus challenge. The data demonstrate that vaccinated animals had a high neutralizing antibody response by 20 days after vaccination and more importantly these animals had a sustained high antibody titer in the early days after viral challenge. On the other hand, the control mice did not have a significant neutralizing antibody titer in the early days after virus infection. The high neutralizing antibody titer in the vaccinated

6

mice was responsible for the lower virus titer in the lungs and consequently resulted in protection against virus infection and the lack of mortality observed.

It is evident from the above results that ozone inactivation provides for an effective way for immunizing mammals against a wide variety of pathogens. Ozone inactivated viruses provided for numerous advantages in that purification of the virus is not required for inactivation. Furthermore, there is no residual chemical inactivant present in the final product. It is found that the inactivated organism has a long storage life under refrigeration, freezing or as a dry lyophilized powder. The inactivation is easily carried out in large amounts under controlled conditions with substantial insurance of effectiveness. The technology is simple, readily reproducible, and the same equipment can be used for a wide variety of viruses.

Although the foregoing invention has been described in some detail by way of illustration and example for purposes of clarity of understanding, it will be obvious that certain changes and modifications may be practiced.

### Claims

1. A vaccine for immunizing a mammalian host which comprises an ozone inactivated but still immunogenic pathogenic virus in a physiologically acceptable carrier.

2. A vaccine according to Claim 1, wherein said microorganism is an enveloped virus.

3. A vaccine according to Claim 1, wherein said virus is an influenza virus.

4. A method for producing a vaccine, which comprises exposing a pathogenic virus in a liquid medium to an ozone containing gas, while maintaining a large surface area contact between said liquid and said gas, at moderate temperature for a time sufficient to inactivate said pathogen without destroying its immugenic property, and isolating said inactivated pathogen and formulating it in a physiologically acceptable medium.

5. A method for producing a vaccine which comprises exposing a pathogenic virus in a liquid medium as a thin film to an ozone containing gas at moderate temperature for a time sufficient to inactivate said pathogen without destroying its immunogenic property, and isolating and formulating said inactivated pathogen in a physiologically acceptable medium.

6. A method according to Claim 5, wherein said thin film is formed by slowing rolling a cylindrical vessel containing a small amount of a nutrient medium as said liquid medium and the ozone concentration is at about 0.1 to 10 ppm.

7. A method for producing a vaccine which comprises exposing a pathogenic virus in an agitated liquid medium to a stream of small bubbles of ozone containing gas at moderate temperature for a time sufficient to inactivate said pathogen without destroying its immunogenic property, isolating said inactivated pathogen and formulating it in a physiologically acceptable medium.

8. A method according to Claim 5, Claim 6 or Claim 7 wherein said exposing is at a temperature in the range of about 10 to 50°C.

9. A method according to any one of Claims 5 to 8 wherein said virus is an enveloped virus.

10. A method according to any one of Claims 5 to 8 wherein said virus is an influenza virus.

11. A vaccine according to any one of Claims 1 to 3 in dosage form, the virus being present in the dose in an amount sufficient to produce an immunogenic response.

### Revendications

1. Vaccin pour immuniser un hôte mammifère qui comprend un virus pathogène inactivé par l'ozone mais encore immunogène dans un véhicule physiologiquement acceptable.

2. Vaccin selon la revendication 1, où ledit micro-organisme est un virus enveloppé.

3. Vaccin selon la revendication 1, où ledit virus est un virus de la grippe.

4. Procédé de production d'un vaccin, qui consiste à exposer un virus pathogène dans un milieu liquide à un gaz contenant de l'ozone, tout en maintenant un contact important de surface entre ledit liquide et ledit gaz, à une température modérée pendant un temps suffisant pour inactiver ledit pathogène, sans détruire sa propriété immunogène et isoler ledit pathogène inactivé et le formuler dans un milieu physiologiquement acceptable.

5. Procédé de production d'un vaccin qui consiste à exposer un virus pathogène dans un milieu liquide sous la forme d'un film mince à un gaz contenant de l'ozone à une température modérée pendant un temps suffisant pour inactiver ledit pathogène sans détruire sa propriété immunogène, et à isoler et à formuler ledit pathogène inactivé dans un milieu physiologiquement acceptable.

6. Procédé selon la revendication 5, caractérisé en ce que ledit film mince est formé en faisant lentement rouler un récipient cylindrique contenant une petite quantité d'un milieu nutritif en tant que milieu liquide et la concentration en ozone est à environ 0,1 à 10 ppm.

7. Procédé de production d'un vaccin qui consiste à exposer un virus pathogène dans un milieu liquide agité à un courant de petites bulles d'ozone contenant du gaz à une température modérée pendant un temps suffisant pour inactiver ledit pathogène sans détruire sa propriété immunogène, à isoler ledit pathogène inactivé et à le formuler dans un milieu physiologiquement acceptable.

8. Procédé selon la revendication 5, la revendication 6 ou la revendication 7, où ladite exposition est à une température comprise entre environ 10 à 50°C.

9. Procédé selon l'une des revendications 5 à 8, où ledit virus est un virus enveloppé.

10. Procédé selon l'une des revendications 5 à 8, où ledit virus est un virus de la grippe.

11. Vaccin selon l'une des revendications 1 à 3, sous forme de dosage, le virus étant présent dans la dose en une quantité suffisante pour produire une réponse immunogène.

**Patentansprüche**

1. Vaccin zur Immunisierung eines Säugetierwirts, das ein durch Ozon inaktiviertes, aber noch immunogen wirksames pathogenes Virus in einem physiologisch annehmbaren Träger enthält.

2. Vaccin nach Anspruch 1, worin der Mikroorganismus ein umhülltes Virus ist.

3. Vaccin nach Anspruch 1, worin das Virus ein Grippevirus ist.

4. Verfahren zur Herstellung eines Vaccins, bei dem ein pathogenes Virus in einem flüssigen Medium einem Ozon enthaltenden Gas unter Aufrechterhaltung eines großflächigen Oberflächenkontakts zwischen der Flüssigkeit und dem Gas bei mittleren Temperaturen während einer Zeit ausgesetzt wird, die ausreicht, um das Pathogen zu inaktivieren, jedoch ohne seine Immunogeneigenschaften zu zerstören, und bei dem das inaktivierte Pathogen abgetrennt und in einem physiologisch annehmbaren Medium formuliert wird.

5. Verfahren zur Herstellung eines Vaccins, bei dem ein pathogenes Virus in einem flüssigen Medium als dünner Film einem ozonhältigen Gas bei mittleren Temperaturen während einer Zeit ausgesetzt wird, die ausreicht, um das Pathogen zu inaktivieren, ohne jedoch seine immunogenen Eigenschaften zu zerstören, und bei dem das inaktivierte Pathogen isoliert und in einem physiologisch annehmbaren Medium formuliert wird.

6. Verfahren nach Anspruch 5, bei dem der dünne Film dadurch gebildet wird, daß ein zylindrisches Gefäß, das eine kleine Menge eines Nährmediums als flüssiges Medium enthält, langsam gerollt wird, und die Ozonkonzentration bei etwa 0,1 bis 10 ppm gehalten wird.

7. Verfahren zur Herstellung eines Vaccins, bei dem ein pathogenes Virus in einem bewegten flüssigen Medium einem Strom von kleinen Bläschen eines ozonhaltigen Gases bei mittlerer Temperatur während einer Zeit ausgesetzt wird, die ausreicht, um das Pathogen zu inaktivieren, ohne jedoch seine immunogenen Eigenschaften zu zerstören, und bei dem das inaktivierte Pathogen abgetrennt und in einem physiologisch annehmbaren Medium formuliert wird.

8. Verfahren nach Anspruch 5, 6 oder 7, bei dem die Behandlung bei einer Temperatur im Bereich von etwa 10 bis 50°C durchgeführt wird.

9. Verfahren nach einem der Ansprüche 5 bis 8, bei dem das Virus ein umhülltes Virus ist.

10. Verfahren nach einem der Ansprüche 5 bis 8, bei dem das Virus ein Grippevirus ist.

11. Vaccin nach einem der Ansprüche 1 bis 3 in einer Dosierungsform, wobei das Virus in der Dosierungsform in einer Menge vorliegt, die ausreicht, um eine Immunogenreaktion hervorzurufen.